# EUROPEAN PATENT APPLICATION

(11) **EP 2 060 268 A1**
(43) Date of publication of application: **20.05.2009**
(21) Application number: 07120813.6
(22) Date of filing: 15.11.2007
(51) Int. Cl.: A61K 38/28, A61K 38/22, A61K 45/06, A61P 3/10, A61P 5/50

(54) **Pharmaceutical compositions for pulmonary or nasal delivery of peptides**

(71) Applicant: NOVO NORDISK A/S, 2880 Bagsvaerd (DK)
(72) Inventor: The designation of the inventor has not yet been filed

(57) **Abstract**

The invention relates to pharmaceutical compositions for pulmonary or nasal delivery of an amylin peptide and an insulin peptide

## Description

### FIELD OF THE INVENTION

The present invention relates to the field of pharmaceutical compositions for treatment of diabetes mellitus and hyperglycaemia. More specifically the invention relates to pharmaceutical compositions for pulmonary or nasal delivery of an amylin peptide and an insulin peptide for systemic absorption to eliminate the need for administering the antidiabetic compounds by injection.

### BACKGROUND OF THE INVENTION

Diabetes mellitus is a metabolic disorder in which the ability to utilize glucose is partly or completely lost. About 5% of all people suffer from diabetes and the disorder approaches epidemic proportions. Since the introduction of insulin in the 1920's, continuous efforts have been made to improve the treatment of diabetes mellitus. Since people suffering from diabetes are subject to chronic treatment over several decades, there is a major need for safe, convenient and life quality improving insulin formulations.

Human insulin consists of two polypeptide chains, the so-called A and B chains which contain 21 and 30 amino acid residues, respectively. The A and B chains are interconnected by two cystine disulphide bridges. Insulin from most other species has a similar construction, but may not contain the same amino acid residues at the same positions. Within the last decade a number of human insulin analogs have been developed. They are designed for particular profiles of action, i.e. fast acting or prolonged action.

In the treatment of diabetes mellitus, many varieties of insulin formulations have been suggested and used, such as regular insulin, isophane insulin (designated NPH), insulin zinc suspensions (such as Semilente^{®}, Lente^{®}, and Ultralente^{®}), and biphasic isophane insulin. Some of the commercial available insulin formulations are characterized by a fast onset of action and other formulations have a relatively slow onset but show a more or less prolonged action. Fast-acting insulin formulations are usually solutions of insulin, while retarded acting insulin formulations can be suspensions containing insulin in crystalline and/or amorphous form precipitated by addition of zinc salts alone or by addition of protamine or by a combination of both.

Normally, insulin formulations are administered by subcutaneous injection. What is important for the patient is the action profile of the insulin formulation which is the action of insulin on the glucose metabolism as a function of the time from the injection. In this profile, inter alia, the time for the onset, the maximum value, and the total duration of action are important. A variety of insulin formulations with different action profiles are desired and requested by the patients.

Currently, the treatment of diabetes, both type 1 diabetes and type 2 diabetes, relies to an increasing extent on the so-called intensive insulin treatment. According to this regimen, the patients are treated with multiple daily insulin injections comprising one or two daily injections of long acting insulin to cover the basal insulin requirement supplemented by bolus injections of rapid acting insulin to cover the insulin requirement related to meals.

Another peptide of interest in the treatment of diabetes is amylin. Amylin is co-secreted with insulin from the pancreatic β-cells in a pulsatile fashion in response to nutrient stimuli. Fasting plasma levels range from 4 to 25 pmol in healthy humans. Typical plasma molar ratio between amylin and insulin is approximately 1:20 (amylin:insulin). This relation could advantageously be mimicked by co-administration of exogenously meal-related insulin and amylin in diabetic patients. However, human amylin is a 37 amino acid long peptide which has physico-chemical properties that make its use as a drug troublesome. In particular, it has a tendency to fibrillate ex-vivo and become ineffective due to precipitation. However, human amylin is a 37 amino acid long peptide which has physico-chemical properties that make its use as a drug troublesome. In particular, it has a tendency to fibrillate ex-vivo and become ineffective due to precipitation. There is currently on the marked a drug product called Symlin containing an analog of human amylin (pramlintide) where the three amino acids in positions 25, 28 and 29 each are substituted to proline. This improves the fibrillating tendency substantially. However, even pramlintide is difficult to keep in solution at neutral pH and it is therefore provided in an acidic solution i.e. Symlin.

The actions of amylin in relation to diabetes are: Reduction of food-intake leading to lower body-weight, slower gastric emptying, smoothening of post-prandial glucose profiles, and reduction in the excessive diabetic glucagon release (A. Young, Amylin: Physiology and Pharmacology, Academic Press (2005)). By and large the actions of amylin are mediated via identified CNS receptors rather than directly on the target organs.

Symlin is approved as an adjunct drug with insulin. Clinical trials have revealed improved HbA1c in the order of 0.3-0.6, a smoother and shallower post-prandial blood glucose profile and reduction in body weight in contrast to treatment with insulin alone. Symlin is currently administered as a separate injection at a separate injection site three times daily. If the patient also uses three insulin injections per day this adds to a total of six daily injections.

Symlin therapy is limited by nausea as a side-effect. The nausea is dose-related but has a tendency to diminish with time. The pharmacokinetic profile of Symlin leads to rather large variations in plasma levels throughout the day. It takes approximately 20 minutes after a subcutaneous injection for Symlin to reach Cₘₐₓ and plasma t½ is in the order of 20 minutes. Ultimately this leads to a need for three or more daily injections of Symlin in order to keep pharmacological plasma level without substantial side-effects. Even with three daily injections Symlin does not mimic the natural release profile of amylin very well. Amylin is released as meal related peaks with a duration close to 3-6 hours in contrast to the 1-1½ hour duration of an injected Symlin profile. Amylin also has a substantial basal level that is not mimicked by Symlin (A. Young, Amylin: Physiology and Pharmacology, Academic Press (2005)).

To date administration of clinically proven peptide hormones such as insulin and Symlin has generally been accomplished by subcutaneous injection which is both inconvenient and unattractive. Therefore, many investigators have studied alternate routes for administering peptide hormones such as oral, pulmonal, rectal, transdermal, and nasal routes.

Not all protein hormones can be efficiently absorbed through the lungs, and there are many factors that affect it. Absorption of proteins in the lung is largely dependent on the physical characteristics of the protein. Efficient pulmonary delivery is furthermore dependent on the ability to deliver the protein to the deep lung alveolar epithelium.

Protein particles that deposite in the upper airway epithelium are not absorbed to a significant extent because the overlying mucus functions to trap, and then clear debris by mucociliary transport up the airway. This mechanism is also a major contributor to low bioavailability. The extent to which proteins are not absorbed and instead eliminated by these routes depends on their solubility, their size, and other largely uncharacterized mechanisms.

Even when a protein can be reproducibly delivered to the deep lung alveolar epithelium, it is difficult to predict whether it will be rapidly absorbed and transported to the blood. Moreover a variety of endogenous peptidases exist in the lung which can degrade peptides prior to absorption. Thus, the longer it takes for a peptide particle to dissolve and be absorbed, the greater the chance for enzymatic inactivation and clearance due to engulfment by macrophages.

It would be useful to provide a pharmaceutical composition for pulmonal or nasal delivery combining administration of an amylin peptide and an insulin peptide in order to be able to administer these peptides to a patient in response to glucose metabolism and thereby limit the number of daily injections or completely replace daily injections.

### SUMMARY OF THE INVENTION

In one aspect the invention relates to a pharmaceutical composition in particulate form comprising an amylin peptide and an insulin peptide. The invention furthermore relates to a method for treatment of hyperglycemia by administering a pharmaceutical composition according to the invention and to a pharmaceutical composition as defined for use as a medicament.

### DESCRIPTION OF THE DRAWINGS

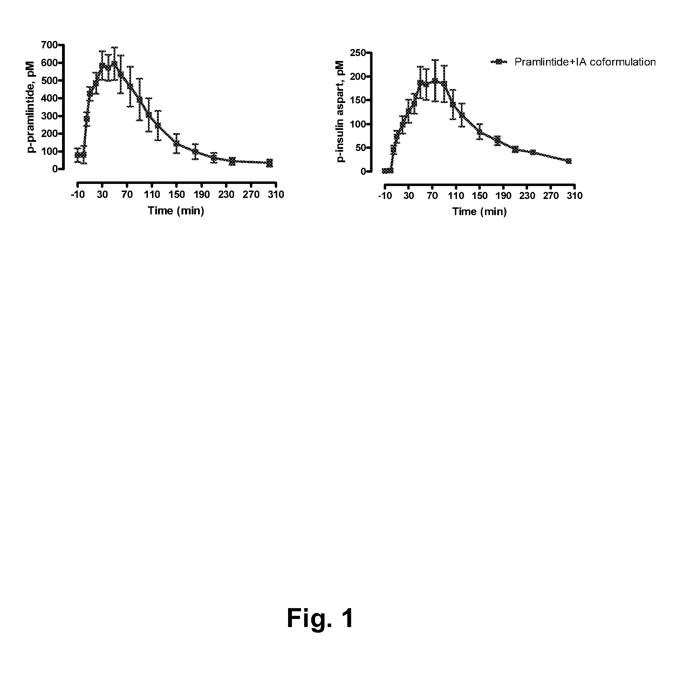

Figure 1: Pharmacokinetic plasma profile of insulin aspart and pramlintide following intratracheal dosing of an insulin aspart and pramlintide dry powder co-formulation.

### DEFINITIONS

The following is a detailed definition of some of the terms used in the specification.

The term "effective amount" as used herein means a dosage which is sufficient in order for the treatment of the patient to be effective compared with no treatment.

The term "medicament" as used herein means a pharmaceutical composition suitable for administration of the pharmaceutically active compounds to a patient.

The term "pharmaceutical composition" as used herein means a product comprising one ore more active peptide(s) optionally together with pharmaceutical excipients such as a buffer, surfactant and bulking agents, said pharmaceutical composition being useful for treating, preventing or reducing the severity of a disease or disorder by administration of said pharmaceutical composition to a person. Thus, a pharmaceutical composition is also known in the art as a pharmaceutical formulation.

The term "pharmaceutically acceptable" as used herein means suited for normal pharmaceutical applications, i.e. giving rise to no serious side effects such as adverse events in patients etc.

The term "stabiliser" as used herein refers to chemicals added to peptide containing pharmaceutical compositions in order to stabilize the peptide(s), i.e. to increase the shelf life and/or in-use time of such compositions. Examples of stabilisers used in pharmaceutical formulations are L-glycine, L-histidine, arginine, polyethylene glycol, and carboxymethylcellulose.

The term "bulking agent" as used herein refers to chemicals added to the peptide containing pharmaceutical compositions in order to increase the volume and mass of a single metered dose, Bulking agents are commonplace in pharmaceutical preparations, especially in formulations for inhalation. They are used to adjust the amount of powder making up unit doses. In general, powder inhalers are capable of delivering a drug substance with good dose accuracy only for certain dose sizes, while different drugs have different potencies and must therefore be delivered in different amounts. As these amounts are often too small for proper dose accuracy to be ensured, bulking agents are added to give the desired dose size. Sugars and sugar alcohols such as as raffinose, melezitose, lactitol, maltitol, trehalose, sucrose, mannitol, inulin and starch may be preferred bulking agent additives for the powders containing polypeptides. A bulking agent may also be useful to obtain an even distribution of two different peptides to be administered in different dosages.

The term "surfactant" as used herein refers to any molecules or ions that are comprised of a water-soluble (hydrophilic) part, the head, and a fat-soluble (lipophilic) segment (the tail) as e.g. described in "Surfactants and Polymers in Aqueous Solution", 2nd ed. by K. Holmberg, B. Lindman et. al.. Surfactants accumulate preferably at interfaces, which the hydrophilic part is orientated towards the water (hydrophilic phase) and the lipophilic part towards the oil- or hydrophobic phase (*i.e.* glass, air, oil etc.). The concentration at which surfactants begin to form micelles is known as the critical micelle concentration or CMC. Furthermore, surfactants lower the surface tension of a liquid. Surfactants are also known as amphipathic compounds. The term "Detergent" is a synonym used for surfactants in general.

The term "aerodynamic diameter" is the diameter of a sphere of unit density (1 g/cc) that has the same gravitational settling velocity as the particle in question. Drug particles for pulmonary delivery are typically characterized by aerodynamic diameter rather than geometric diameter. The velocity at which the drug settles is proportional to the aerodynamic diameter, dₐ.

dₐ = (p/X)1/2 * dg where ρ = density and X = shape factor, dg = geometric diameter For spherical species, X = 1.

For nasal, bronchial and pulmonary drug delivery the mass median aerodynamic diameter of the particles administered determines the place of deposition. Because of the large difference in size required unique particle formation processes often has to be developed if a drug has to be tested for more than one route of delivery. E.g. for pulmonary delivery a mass mean aerodynamic diameter (MMAD) of 1-5 µm is normally considered optimal and for nasal delivery MMAD above 10 µm is normally considered optimal.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

The term "analog" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been added to the peptide. In one aspect of the invention, such addition or deletion of amino acid residues can take place at the N-terminal of the peptide and/or at the C-terminal of the peptide. In one embodiment an analog comprises less than 8 modifications (substitutions, deletions, additions) relative to the native peptide. In one embodiment an analog comprises less than 7 modifications (substitutions, deletions, additions) relative to the native peptide. In one embodiment an analog comprises less than 6 modifications (substitutions, deletions, additions) relative to the native peptide. In another embodiment an analog comprises less than 5 modifications (substitutions, deletions, additions) relative to the native peptide. In another embodiment an analog comprises less than 4 modifications (substitutions, deletions, additions) relative to the native peptide. In another embodiment an analog comprises less than 3 modifications (substitutions, deletions, additions) relative to the native peptide. In another embodiment an analog comprises less than 2 modifications (substitutions, deletions, additions) relative to the native peptide. In another embodiment an analog comprises only a single modification (substitutions, deletions, additions) relative to the native peptide.

In one embodiment, the modification(s) are substitution(s). In another embodiment, the modification(s) are deletions(s). In another embodiment, the modification(s) are addition(s).

The term "derivative" as used herein in relation to a peptide means a chemically modified parent peptide or an analog thereof, wherein at least one substituent is not present in the parent peptide or an analog thereof, i.e. a parent peptide which has been covalently modified. Typical modifications are amides, carbohydrates, alkyl groups, acyl groups, esters, PEGylations and the like. Examples of derivatives of human insulin are threonine methyl ester^{B30} human insulin and N^{εB29}-tetradecanoyl des(B30) human insulin.

With "insulin peptide" as used herein is meant human insulin, porcine insulin or bovine insulin with disulfide bridges between CysA7 and CysB7 and between CysA20 and CysB19 and an internal disulfide bridge between CysA6 and CysA11 or an insulin analog or a derivative thereof.

The term "amylin peptide" as used herein means a peptide which is amylin, an analog or derivative thereof or an amylin agonist. It is understood that biological active amylin agonists may have an amide group attached to the acid group of the C terminal residue via a peptide bond.

Analogs of e.g. human amylin (h-amylin) may be denoted by the changed position or positions in superscript followed by the three letter code for the amino acid substituting the native amino acid or amino acids if several residues are changed to the same amino acid. Thus, pramlintide may be denoted as ^{25,28,29}Pro-h-amylin indicating that the positions 25, 28 and 29 in human amylin all have been changed to proline. By "des-¹Lys" is meant that the lysine in position 1 is lacking, e.g. des-¹Lys¹⁸Arg^{25, 28}Pro-h-amylin is a human amylin analog where the lysine in position 1 is lacking, the amino acid in position 18 is changed to an arginine, and the two amino acids in positions 25 and 28 are each changed to a proline.

The term "isoelectric point" as used herein means the pH value where the overall net charge of a macromolecule such as a peptide is zero. In peptides there may be several charged groups, and at the isoelectric point the sum of all these charges is zero. At a pH above the isoelectric point the overall net charge of the peptide will be negative, whereas at pH values below the isoelectric point the overall net charge of the peptide will be positive.

The term "about" as used herein in relation to the concentration of a peptide in a pharmaceutical composition means plus or minus 10%. Hence, the concentration "about 5 mg/mL insulin" means a concentration of 4.5 mg/mL insulin to 5.5 mg/mL insulin. In one embodiment, where the term "about" is used the corresponding value or range includes the exact value or range as if the term was not present.

A "peptide solution" as mentioned herein means a solution of one or more polypeptides which has been solubilised without the use of acids and at pH values above the isoelectric point of the polypeptide.

By "volatile base" is meant a base, which to some extend will evaporate upon heating and/or at reduced pressure, e.g. bases which have a vapour pressure above 65 Pa at room temperature or an aqueous azeotropic mixture including a base having a vapour pressure above 65 Pa at room temperature .

By "volatile acid" is meant a acid, which to some extend will evaporate upon heating and/or at reduced pressure, e.g. acids which have a vapour pressure above 65 Pa at room temperature or an aqueous azeotropic mixture including a acid having a vapour pressure above 65 Pa at room temperature .

A "non volatile base" as mentioned herein means a base, which do not evaporate or only partly evaporate upon heating, e.g. bases with a vapour pressure below 65 Pa.

A "non volatile acid" as mentioned herein means a acid, which do not evaporate or only partly evaporate upon heating, e.g. acids with a vapour pressure below 65 Pa.

By "pH of the peptide solution/insulin solution/amylin solution" is meant the pH of the peptide/insulin/amylin solution after adjustment with volatile and non volatile bases and before spray drying of the peptide solution.

By "pH of the spray dried peptide/insulin/amylin" is meant the pH of the spray dried peptide/insulin/amylin when mixing approximately 20 mg spray dried peptide with 0.5 ml demineralised water.

### DESCRIPTION OF THE INVENTION

It has now been found that it is desirable to combine an amylin peptide and an insulin peptide in particulate form for pulmonal or nasal delivery to improve the treatment of diabetes. Amylin is a hormone which is secreted with insulin from the pancreatic 0-cells in normal (non-diabetic) individuals. It is believed that amylin modulates insulin activity in vivo, and it has been proposed that simultaneous administration of amylin with insulin could improve blood glucose control. Combining a dry powder amylin peptide with insulin peptide provides in a composition for pulmonal or nasal delivery a particularly convenient product for achieving simultaneous administration. An amylin peptide such as amylin may be combined with an insulin peptide such as insulin at from 0.1 % by weight to 10% by weight (based on the total weight of insulin in a dose), preferably from 0.5% by weight to 2.5% by weight. Amylin is available from commercial suppliers, such as Amylin Corporation, San Diego, California, and can be readily formulated together with an insulin peptide in the compositions of the present invention. For example, an amylin peptide may be dissolved in aqueous or other suitable solutions together with the insulin peptide, and optionally carriers and excipients, and the solution spray dried to produce a powder product.

In one aspect of the invention, a pharmaceutical composition in particulate form comprising an amylin peptide and an insulin peptide is provided. In one aspect of the invention, the pharmaceutical composition in particulate form comprises the amylin peptide and the insulin peptide in a co-formulated form. In a further aspect of the invention, the pharmaceutical composition in particulate form comprises the insulin peptide and the amylin peptide formulated as two separate powders. In a further aspect of the invention, when the peptides are in the form of two separate powders they may be co-administered in a device with a double chamber design. In yet a further aspect of the invention, when the peptides are in the form of two separate powders they may be administered as a physical mixture of the two powders.

The pharmaceutical composition for oral inhalation according to the invention has in one aspect a mean aerodynamic diameter of 0.5 to 10 µm, preferred a mean aerodynamic diameter of 1 to 5 µm, more preferred a mean aerodynamic diameter of 1 to 4 µm and most preferred a mean aerodynamic diameter of 1.5 to 3 µm. For nasal delivery the pharmaceutical composition according to the invention has in one aspect a mean aerodynamic diameter above 10 µm. The mean aerodynamic diameter is measured by cascade impactors such as the Next Generation Impactors (NGI), time of flight instruments, such as the Aerodynamic Particle Sizer, whereas the geometric diameter can be measured with e.g. laser diffractions instruments.

In another aspect of the invention, a process for preparing a pharmaceutical composition in the form of a co-formulation by spray drying or freeze drying of a solution containing both an insulin peptide and an amylin peptide to obtain a dry powder, and optionally comminution, is provided.

In another aspect of the invention, a process for preparing a pharmaceutical composition in the form of a physical mixture of a powder containing and insulin peptide and a powder containing an amylin peptide by spray drying or freeze drying independently of a solution containing an insulin peptide and a solution containing an amylin peptide and mixing the two powders, is provided. In one aspect, the mixing is obtained by a turbula mixer.

An insulin analog as used herein is a polypeptide which has a molecular structure which formally can be derived from the structure of a naturally occurring insulin, for example that of human insulin, by deleting and/or substituting at least one amino acid residue occurring in the natural insulin and/or by adding at least one amino acid residue.

The insulin analogs may be such wherein position 28 of the B chain may be modified from the natural Pro residue to one of Asp, Lys, Leu, Val, Ala or Ile. In another aspect Lys at position B29 of insulin is modified to Pro or Glu. In one aspect an insulin analog according to the invention is such wherein the amino acid residue in position B28 of insulin is Pro, Asp, Lys, Leu, Val, or Ala and the amino acid residue in position B29 is Lys or Pro and optionally the amino acid residue in position B30 is deleted. Also, Asn at position A21 may be modified to Ala, Gin, Glu, Gly, His, Ile, Leu, Met, Ser, Thr, Trp, Tyr or Val, in particular to Gly, Ala, Ser, or Thr and preferably to Gly. Furthermore, Asn at position B3 may be modified to Lys,Thr, Ser, Gln, Glu or Asp. Further examples of insulin analogs are des(B30) human insulin; des(B30) human insulin analogs; insulin analogs wherein PheB1 has been deleted; insulin analogs wherein the A-chain and/or the B-chain have an N-terminal extension and insulin analogs wherein the A-chain and/or the B-chain have a C-terminal extension. Thus one or two Arg may be added to position B1. In another aspect an insulin analog according to the invention is des(B28-B30) human insulin, des(B27) human insulin or des(B30) human insulin. In yet another aspect an insulin analog according to the invention is an insulin analog wherein the amino acid residue in position B3 is Lys and the amino acid residue in position B29 is Glu or Asp.

In another aspect an insulin analog according to the invention is des(B28-B30) human insulin, des(B27) human insulin or des(B30) human insulin. In yet another aspect an insulin analog according to the invention is an insulin analog wherein the amino acid residue in position B3 is Lys and the amino acid residue in position B29 is Glu or Asp.

The term "human insulin" as used herein means the human hormone whose structure and properties are well-known. Human insulin has two polypeptide chains that are connected by disulphide bridges between cysteine residues, namely the A-chain and the B-chain. The A-chain is a 21 amino acid peptide and the B-chain is a 30 amino acid peptide, the two chains being connected by three disulphide bridges: one between the cysteines in position 6 and 11 of the A-chain, the second between the cysteine in position 7 of the A-chain and the cysteine in position 7 of the B-chain, and the third between the cysteine in position 20 of the A-chain and the cysteine in position 19 of the B-chain.

Mutations in the insulin molecule are denoted in superscript stating the chain (A or B), the position, and the single letter code for the amino acid substituting the native amino acid. By "desB30" is meant a natural insulin B chain or an analog thereof lacking the B30 amino acid. Thus, human insulin ^{A21G, B28D, desB30} is an analog of human insulin where position 21 in the A chain is mutated to glycine, position 28 in the B chain is mutated to aspartic acid, and position 30 in the B chain is deleted. By "parent insulin" is meant a naturally occurring insulin such as human insulin or porcine insulin. Alternatively, the parent insulin can be an insulin analog.

In one aspect of the invention, the insulin peptide is human insulin, an analog of human insulin, a derivative of human insulin or a derivative of a human insulin analog.

In one aspect of the invention, the insulin peptide is human insulin.

In one aspect of the invention, the insulin peptide is an insulin derivative. In a further aspect of the invention, the insulin derivative is selected from the group consisting of B29-N^{ε} myristoyl-des(B30) human insulin, B29-N^{ε}-palmitoyl-des(B30) human insulin, B29-N^{ε}-myristoyl human insulin, B29-N^{ε}-palmitoyl human insulin, B28-N^{ε}-myristoyl Lys^{B28} Pro^{B29} human insulin, B28-N^{ε}-palmitoyl Lys^{B28} Pro^{B29} human insulin, B30-N^{ε}-myristoyl-Thr^{B29}Lys^{B30} human insulin, B30-N^{ε}-palmitoyl-Thr^{B29}Lys^{B30} human insulin, B29-N^{ε}-(N-palmitoyl-γ-glutamyl)-des(B30) human insulin, B29-N^{ε}-(N-lithocholyl-γ-glutamyl)-des(B30) human insulin, B29-N^{ε}-(ω-carboxyheptadecanoyl)-des(B30) human insulin and B29-N^{ε}-(ω-carboxyheptadecanoyl) human insulin.

In another aspect of the invention, the insulin derivative is B29-N^{ε}-myristoyldes(B30) human insulin.

In a further aspect of the invention the insulin peptide is acid-stabilised insulin.

The acid-stabilised insulin may be selected from analogs of human insulin having one of the following amino acid residue substitutions:
A21 G
A21G, B28K, B29P
A21G, B28D
A21G, B28E
A21G, B3K, B29E
A21G, desB27
A21G, B9E
A21G, B9D
A21G, B10E insulin.

In a further aspect of the invention, the insulin peptide is an insulin analog. The insulin analog may be selected from the group consisting of an analog wherein position B28 is Asp, Lys, Leu, Val, or Ala and position B29 is Lys or Pro; and des(B28-B30), des(B27) or des(B30) human insulin.

In another aspect of the invention, the insulin analog is an analog of human insulin wherein position B28 is Asp or Lys, and position B29 is Lys or Pro.

In another aspect of the invention, the insulin analog is des(B30) human insulin.

In another aspect of the invention, the insulin analog is an analog of human insulin wherein position B28 is Asp.

In another aspect of the invention, the insulin analog is an analog wherein position B3 is Lys and position B29 is Glu or Asp.

In another aspect of the invention, the insulin analogs and derivatives are selected from among those disclosed in EP 0 792 290 (Novo Nordisk A/S), EP 0 214 826 and EP 0 705 275 (Novo Nordisk A/S), US 5,504,188 (Eli Lilly), EP 0 368 187 (Aventis), US patents 5,750,497 and 6,011,007, EP 375437 and EP 383472 and where such insulins may include, but are not limited to, insulin glulisine ( also known as Apidra®, differs from human insulin in that the amino acid asparagine at position B3 is replaced by lysine and the lysine in position B29 is replaced by glutamic acid), Lys^{B28} Pro^{B29} human insulin (Humalog®) , and Asp^{B28} human insulin (insulin aspart (Novolog®)).

In one aspect of the invention, said human insulin analog is Asp^{B28}-human insulin. In another aspect of the invention, said human insulin analog is Lys^{B28},Pro^{B29}-human insulin. In another aspect of the invention, said human insulin analog is Lys^{B3},Glu^{B29}-human insulin (insulin glulisine). In another aspect of the invention, said human insulin analog is des(B30) human insulin.

Also, derivatives of precursors or intermediates are covered by the invention. An example of such a derivative is a single-chain insulin which comprises the B- and the A-chain of human insulin or analogs or derivatives thereof connected by a connecting peptide.

An insulin derivative according to the invention is a naturally occurring insulin or an insulin analog which has been chemically modified, e.g. by introducing a side chain in one or more positions of the insulin backbone or by oxidizing or reducing groups of the amino acid residues in the insulin or by converting a free carboxylic group to an ester group or to an amide group. Other derivatives are obtained by acylating a free amino group or a hydroxy group, such as in the B29 position of human insulin or desB30 human insulin. A non-limiting example of acylated polypeptides may e.g. be found in WO 95/07931 which is are hereby incorporated by reference.

The term "meal-related insulin peptide" as used herein means an insulin peptide which has a time-action of less than 8 hours in standard models of diabetes. e.g. pharmacokinetic disappearance and/or appearance" in pigs.

Preferably, the meal-related human insulin has a time-action of less than about 5 hours. Preferably, the meal-related insulin has a time-action in the range from 0 hours to about 4 hours. Preferably, the meal-related insulin has a time-action similar to that observed for commercial pharmaceutical compositions of Actrapid^{®}, Novolog^{®}, Humalog^{®} and Apidra^{®}. The term about in relation to the time-action of insulins means + or - 30 minutes

In another embodiment of the invention, the pharmaceutical composition comprises two different insulin peptides.

In one aspect of the invention, the amylin peptide is amylin, an amylin analog or an amylin agonist.

"Amylin" as used herein refers to compounds such as those described in U. S. Patents 5,124,314 and 5,234, 906, both of which are hereby incorporated by reference. The term includes, but is not limited to, a human peptide hormone of 37 amino acids referred to as amylin, which is co-secreted with insulin from β-cells of the pancreas. Human amylin has the following amino acid sequence: Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr (SEQ ID NO:1). Thus, the structural formula is Lys-Cys-Asn-Thr-Ala-Thr-Cys-Ala-Thr-Gln-Arg-Leu-Ala- Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr-NH₂ (SEQ ID NO: 1) with a disulfidebridge between the two Cys residues and an amide group attached to the C-terminal amino acid via a peptide bond. The term also includes variants of amylin as present in, and in isolatable form, other mammalian species. With respect to a naturally occurring amylin compound, the term includes such a compound in an isolated, purified, or other form that is otherwise not found in nature.

An "agonist" of amylin refers to a compound that mimics one or more effects (or activity) of amylin *in vitro* or *in vivo.* The effects of amylin include the ability to directly or indirectly interact or bind with one or more receptors that are activated or deactivated by amylin, for example, the receptor binding assay and the soleus muscle assay described in Examples 2 and 3, respectively in WO 2004/037168. Preferably, the amylin agonist is not a calcitonin, which, as used herein, refers to the human peptide hormone calcitonin and species variations of it, such as that of rat, salmon 10 and eel (including aminosuberic eel calcitonin).

An "analog" (or "analog" or "agonist analog") of amylin refers to a compound that is similar in structure (e.g., derived from the primary amino acid sequence of amylin by substituting one or more natural or unnatural amino acids or peptidomimetics) to amylin and mimics an effect of amylin *in vitro* or *in vivo.* Amylin analogs useful according to the invention may also include fragments of amylin such as those described in EP 289287, the contents of which are herein incorporated by reference. Preferred amylin agonists may also be compounds having at least 60, 65, 70, 75, 80, 85, 90, 95, or 99% amino acid sequence identity to SEQ ID NO: 1 and having amylin activity. The amylin peptide of the present invention may be capable of binding to or otherwise directly or indirectly interacting with an amylin receptor, or other receptor or receptors with which amylin itself may interact to elicit a biological response, e.g., reducing food intake. Compounds of the invention may bind an amylin receptor with an affinity of greater than 20 nM, 10 nM, 5 nM, and more preferably with an affinity of greater than 0.10 nM e.g. as determined by the amylin receptor assay in the section "Methods".

Amylin analogs also include amylin having insertions, deletions, and/or substitutions in at least one or more amino acid positions of SEQ ID NO: 1. The number of amino acid insertions, deletions, or substitutions may be at least 1, 2, 3, 4, 5, 6, 10, 15, 20, 25, or 30. Insertions or substitutions may be with other natural or unnatural amino acids, synthetic amino acids, peptidomimetics, or other chemical compounds.

A "derivative" of amylin refers to an amylin which is chemically modified such as N-methylated amylin. In one aspect of the invention, the amylin peptide is amylin N-methylated in positions 24 and 26 as described in Yan et al, PNAS, vol. 103, no. 7, p. 2046-2051, 2006.

Exemplary amylin agonist analogs contemplated in the use of the invention include those described in U.S. Patent Nos. 5,686,411, 6,114, 304, and 6,410,511, which are herein incorporated by reference in their entirety. In a further aspect the amylin derivatives contemplated in the use of the invention include those described in DE102004051014.

Exemplary compounds include, but are not limited to des-¹Lys-h-amylin, ²⁸Pro-h-amylin , ^{25, 28,29}Pro-h-amylin, ¹⁸Arg^{25, 28}Pro-h-amylin, and des-¹Lys¹⁸Arg^{25, 28}Pro-h-amylin, all show amylin activity *in vivo* in treated test animals, (e.g. provoking marked hyperlactemia followed by hyperglycemia). In addition to having activities characteristic of amylin, certain of the preferred compounds of the invention have also been found to possess more desirable solubility and stability characteristics when compared to human amylin. Examples of these compounds include ²⁵Pro²⁶Val^{28, 29}Pro-h-amylin, ^{25, 28, 29}Pro-h-amylin, and ¹⁸Arg^{25, 28}Pro-h- amylin.

In one aspect of the invention, said amylin peptide is human amylin. In one aspect of the invention, said amylin peptide is ^{25,28,29}Pro- h-amylin (pramlintide). In a further aspect of the invention, said amylin peptide is human amylin methylated in position 24 and 26.

In one aspect of the invention, said meal-related insulin peptide is human insulin and said amylin peptide is ^{25, 28, 29}Pro- h-amylin. In a further aspect of the invention, said insulin and amylin formulated as dry powders is mixed in an amylin:insulin molar ratio between about 1:99 to about 10:1.

In another aspect of the invention, said insulin peptide is human insulin^{B28D} and said amylin peptide is ^{25, 28, 29}Pro- h-amylin. In a further aspect of the invention, said insulin and amylin formulated as dry powders is mixed in an amylin:insulin molar ratio between about 1:99 to about 10:1.

In another aspect of the invention, said insulin peptide is human insulin^{B3K, B29E} and said amylin peptide is ^{25, 28, 29}Pro- h-amylin. In a further aspect of the invention, said insulin and amylin formulated as dry powders is mixed in an amylin:insulin molar ratio between about 1:99 to about 10:1.

In another aspect of the invention, said insulin peptide is human insulin^{B28K, B29P} and said amylin peptide is ^{25,28,29}Pro- h-amylin. In a further aspect of the invention, the said insulin and amylin formulated as dry powders is mixed in an amylin:insulin molar ratio between about 1:99 to about 10:1.

In one aspect, the present invention relates to a pharmaceutical composition which further comprises zinc and/or calcium. In one aspect, the present invention relates to a pharmaceutical composition which further comprises zinc. In another aspect, the present invention relates to a pharmaceutical composition which further comprises calcium. In one embodiment of the invention, the molar ratio of zinc to insulin peptide is from 1/6 to ½ mole/mole, preferable from 3/12 to 5/12 mole/mole. In a further embodiment of the invention, the molar ratio of calcium to insulin peptide is from 1/12 to 5/12 mole/mole, preferable from 1/6 to 1/3 mole/mole, such as from 3/12 to 5/12 mole/mole.

In another embodiment, the physical stability of the insulin and/or the amylin peptide(s) in a composition of the invention can be determined by a ThT fibrillation assay for the assessment of physical stability of peptide formulations.

In one embodiment of the invention, the pharmaceutical composition according to the invention is a "physical stable" pharmaceutically composition. The term "physical stable" as used in this context means that the amylin peptide and the insulin peptide does not destabilize each other in the combined composition i.e. the pharmaceutical composition is as physical stable as the least stable of the amylin peptide and the insulin peptide alone. Physical stability may be determined as described in the Thioflavine T (ThT) fibrillation assay by Nielsen et al. (2001) Biochemistry 40, 6036-6046.

In a further embodiment of the invention, said pharmaceutical composition comprises a stabiliser. In one embodiment, said stabiliser is selected from the group consisting of glass stabiliser such as mannitol, sucrose, lactose, trehalose, inulin, or L-histidine, imidazole and L-leucine. In one embodiment, said stabiliser is a polyethylene glycol.

In a further embodiment of the invention, said pharmaceutical composition comprises a surfactant. As examples of surfactants mention can be made of anionic surfactants, cationic surfactants, nonionic surfactants, and zwitterionic surfactants.

In one aspect of the invention, the surfactant is an anionic surfactant.

In one aspect of the invention, the pharmaceutical composition comprises an anionic surfactant in a concentration higher than its critical micelle concentration (CMC).

Anionic surfactants may be selected from the group of: Chenodeoxycholic acid, Chenodeoxycholic acid sodium salt, Cholic acid, Dehydrocholic acid, Deoxycholic acid, Deoxycholic acid methyl ester, Digitonin, Digitoxigenin, N,N-Dimethyldodecylamine N-oxide, Docusate sodium, Glycochenodeoxycholic acid sodium, Glycocholic acid hydrate, Glycodeoxycholic acid monohydrate, Glycodeoxycholic acid sodium salt, Glycodeoxycholic acid sodium salt, Glycolithocholic acid 3-sulfate disodium salt, Glycolithocholic acid ethyl ester, N-Lauroylsarcosine sodium salt, N-Lauroylsarcosine sodium salt, N-Lauroylsarcosine, N-Lauroylsarcosine, Lithium dodecyl sulfate, Lugol, 1-Octanesulfonic acid sodium salt, 1-Octanesulfonic acid sodium salt, Sodium 1-butanesulfonate, Sodium 1-decanesulfonate, Sodium 1-dodecanesulfonate, Sodium 1-heptanesulfonate, Sodium 1-heptanesulfonate, Sodium 1-nonanesulfonate, Sodium 1-propanesulfonate monohydrate, Sodium 2-bromoethanesulfonate, Sodium cholate hydrate, ox or sheep bile, Sodium cholate hydrate, Sodium choleate, Sodium deoxycholate, Sodium dodecyl sulfate, Sodium dodecyl sulfate, Sodium hexanesulfonate, Sodium octyl sulfate, Sodium pentanesulfonate, Sodium taurocholate, Taurochenodeoxycholic acid sodium salt, Taurodeoxycholic acid sodium salt monohydrate, Taurolithocholic acid 3-sulfate disodium salt, Tauroursodeoxycholic acid sodium salt, Trizma^{®} dodecyl sulfate, DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), Dodecylphosphocholine (FOS-Choline-12), Decylphosphocholine (FOS-Choline-10), Nonylphosphocholine (FOS-Choline-9), dipalmitoyl phosphatidic acid, sodium caprylate, and/or Ursodeoxycholic acid.

In one aspect of the invention, the anionic surfactant is a glycerophosphoglycerol derivative. In one aspect of the invention, said glycerophosphoglycerol derivative is a dimyristoyl derivative, e.g. 1,2-Dimyristoyl-*sn*-glycero-3-phospho-*rac*-(1-glycerol). In one aspect of the invention, the dimyristoyl derivative is added in a concentration between 0.1 mM and 10 mM, preferably between 0.5 mM and 5 mM, preferably between 0.5 mM and 3 mM. In one aspect of the invention, said glycerophosphoglycerol derivative is 1,2-Dimyristoyl-sn-glycero-3-phospho-rac-(1-glycerol) (DMPG), such as CAS no. 67232-80-8.

In one aspect of the invention, the surfactant is a cationic surfactant.

Cationic surfactants may be selected from the group of: Alkyltrimethylammonium bromide, Benzalkonium chloride, Benzalkonium chloride, Benzyldimethylhexadecylammonium chloride, Benzyldimethyltetradecylammonium chloride, Benzyltrimethylammonium tetrachloroiodate, Dimethyldioctadecylammonium bromide, Dodecylethyldimethylammonium bromide, Dodecyltrimethylammonium bromide, Dodecyltrimethylammonium bromide, Ethylhexadecyldimethylammonium bromide, Hexadecyltrimethylammonium bromide, Hexadecyltrimethylammonium bromide, Polyoxyethylene(10)-N-tallow-1,3-diaminopropane, Thonzonium bromide, and/or Trimethyl(tetradecyl)ammonium bromide.

In one aspect of the invention, the surfactant is a nonionic surfactant.

Nonionic surfactants may be selected from the group of: BigCHAP, Bis(polyethylene glycol bis[imidazoyl carbonyl]), block copolymers as polyethyleneoxide/polypropyleneoxide block copolymers such as poloxamers, poloxamer 188 and poloxamer 407, Brij^{®} 35, Brij^{®} 56, Brij^{®} 72, Brij^{®} 76, Brij^{®} 92V, Brij^{®} 97, Brij^{®} 58P, Cremophor^{®} EL, Decaethylene glycol monododecyl ether, N-Decanoyl-N-methylglucamine, n-Dodecanoyl-N-methylglucamide, alkyl-polyglucosides, ethoxylated castor oil, Heptaethylene glycol monodecyl ether, Heptaethylene glycol monododecyl ether, Heptaethylene glycol monotetradecyl ether, Hexaethylene glycol monododecyl ether, Hexaethylene glycol monohexadecyl ether, Hexaethylene glycol monooctadecyl ether, Hexaethylene glycol monotetradecyl ether, Igepal CA-630, Igepal CA-630, Methyl-6-O-(N-heptylcarbamoyl)-beta-D-glucopyranoside, Nonaethylene glycol monododecyl ether, N-Nonanoyl-N-methylglucamine, N-Nonanoyl-N-methylglucamine, Octaethylene glycol monodecyl ether, Octaethylene glycol monododecyl ether, Octaethylene glycol monohexadecyl ether, Octaethylene glycol monooctadecyl ether, Octaethylene glycol monotetradecyl ether, Octyl-β-D-glucopyranoside, Pentaethylene glycol monodecyl ether, Pentaethylene glycol monododecyl ether, Pentaethylene glycol monohexadecyl ether, Pentaethylene glycol monohexyl ether, Pentaethylene glycol monooctadecyl ether, Pentaethylene glycol monooctyl ether, Polyethylene glycol diglycidyl ether, Polyethylene glycol ether W-1, Polyoxyethylene 10 tridecyl ether, Polyoxyethylene 100 stearate, Polyoxyethylene 20 isohexadecyl ether, Polyoxyethylene 20 oleyl ether, Polyoxyethylene 40 stearate, Polyoxyethylene 50 stearate, Polyoxyethylene 8 stearate, Polyoxyethylene bis(imidazolyl carbonyl), Polyoxyethylene 25 propylene glycol stearate, Saponin from Quillaja bark, Span^{Ⓡ} 20, Span^{Ⓡ} 40, Span^{Ⓡ} 60, Span^{Ⓡ} 65, Span^{Ⓡ} 80, Span^{Ⓡ} 85, Tergitol, Type 15-S-12, Tergitol, Type 15-S-30, Tergitol, Type 15-S-5, Tergitol, Type 15-S-7, Tergitol, Type 15-S-9, Tergitol, Type NP-10, Tergitol, Type NP-4, Tergitol, Type NP-40, Tergitol, Type NP-7, Tergitol, Type NP-9, Tetradecyl-β-D-maltoside, Tetraethylene glycol monodecyl ether, Tetraethylene glycol monododecyl ether, Tetraethylene glycol monotetradecyl ether, Triethylene glycol monodecyl ether, Triethylene glycol monododecyl ether, Triethylene glycol monohexadecyl ether, Triethylene glycol monooctyl ether, Triethylene glycol monotetradecyl ether, Triton CF-21, Triton CF-32, Triton DF-12, Triton DF-16, Triton GR-5M, Triton QS-15, Triton QS-44, Triton X-100, Triton X-1 02, Triton X-15, Triton X-151, Triton X-200, Triton X-207, Triton^{Ⓡ} X-1 00, Triton^{Ⓡ} X-114, Triton^{Ⓡ} X-165 solution, Triton^{Ⓡ} X-305 solution, Triton^{Ⓡ} X-405, Triton^{Ⓡ} X-45, Triton^{Ⓡ} X-705-70, TWEEN^{Ⓡ} 20, TWEEN^{Ⓡ} 40, TWEEN^{Ⓡ} 60, TWEEN^{Ⓡ} 6, TWEEN^{Ⓡ} 65, TWEEN^{®} 80, TWEEN^{Ⓡ} 81, TWEEN^{Ⓡ} 85, Tyloxapol, sphingophospholipids (sphingomyelin), Solutol HS 15 (also known as Macrogol 15 Hydroxystearate) and d-alfa-Tocopheryl polyethylene glycol 1000 succinate (also known as vitamin E TPGS or TPGS), and sphingoglycolipids (ceramides, gangliosides), phospholipids, and/or n-Undecyl β-D-glucopyranoside.

In one aspect of the invention, the surfactant is a zwitterionic surfactant.

Zwitterionic surfactants may be selected from the group of: CHAPS, CHAPSO, 3-(Decyldimethylammonio)propanesulfonate inner salt, 3-(Dodecyldimethylammonio)-propanesulfonate inner salt, 3-(Dodecyldimethylammonio)propanesulfonate inner salt, 3-(N,N-Dimethylmyristylammonio)propanesulfonate, 3-(N,N-Dimethyloctadecylammonio)-propanesulfonate, 3-(N,N-Dimethyloctylammonio)propanesulfonate inner salt, 3-(N,N-Dimethylpalmitylammonio)propanesulfonate, N-alkyl-N,N-dimethylammonio-1-propanesulfonates, 3-cholamido-1-propyldimethylammonio-1-propanesulfonate, Dodecylphosphocholine, myristoyl lysophosphatidylcholine, Zwittergent 3-12 (*N*-dodecyl-*N,N-*dimethyl-3-ammonio-1-propanesulfonate), Zwittergent 3-10 (3-(Decyldimethylammonio)-propanesulfonate inner salt), Zwittergent 3-08 (3-(Octyldimethylammonio)pro-panesulfonate), glycerophospholipids (lecithins, kephalins, phosphatidyl serine), glyceroglycolipids (galactopyranoside), alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, lysophosphatidylserine and lysophosphatidylthreonine, acylcarnitines and derivatives, N^{beta}-acylated derivatives of lysine, arginine or histidine, or side-chain acylated derivatives of lysine or arginine, N^{beta}-acylated derivatives of dipeptides comprising any combination of lysine, arginine or histidine and a neutral or acidic amino acid, N^{beta}-acylated derivative of a tripeptide comprising any combination of a neutral amino acid and two charged amino acids, or the surfactant may be selected from the group of imidazoline derivatives, long-chain fatty acids and salts thereof C₆-C₁₂ (eg. oleic acid and caprylic acid), N-Hexadecyl-N,N-dimethyl-3-ammonio-1-propanesulfonate, anionic (alkyl-aryl-sulphonates) monovalent surfactants, palmitoyl lysophosphatidyl-L-serine, lysophospholipids (e.g. 1-acyl-sn-glycero-3-phosphate esters of ethanolamine, choline, serine or threonine), or mixtures thereof.

In one aspect of the invention, the surfactant is an alkyl-polyglucoside. The term "alkyl-polyglucosides" as used herein in relates to a straight or branched C₅₋₂₀-alkyl, -alkenyl or - alkynyl chain which is substituted by one or more glucoside moieties such as maltoside, saccharide etc. Embodiments of these alkyl-polyglucosides include C₆₋₁₈-alkyl-polyglucosides. Specific embodiments of these alkyl-polyglucosides includes the even numbered carbon-chains such as C₆, C₈, C₁₀, C₁₂, C₁₄, C₁₆, C₁₈ and C₂₀ alkyl chain. Specific embodiments of the glucoside moieties include pyranoside, glucopyranoside, maltoside, maltotrioside and sucrose. In embodiments of the invention, less than 6 glucosid moieties are attached to the alkyl group. In embodiments of the invention, less than 5 glucosid moieties are attached to the alkyl group. In embodiments of the invention, less than 4 glucosid moieties are attached to the alkyl group. In embodiments of the invention, less than 3 glucosid moieties are attached to the alkyl group. In embodiments of the invention, less than 2 glucosid moieties are attached to the alkyl group. Specific embodiments of alkyl-polyglucosides are alkyl glucosides such n-decyl β-D-glucopyranoside, decyl β-D-maltopyranoside, dodecyl β-D-glucopyranoside, n-dodecyl β-D-maltoside, tetradecyl β-D-glucopyranoside, decyl β-D-maltoside, hexadecyl β-D-maltoside, decyl β-D-maltotrioside, dodecyl β-D-maltotrioside, tetradecyl β-D-maltotrioside, hexadecyl β-D-maltotrioside, n-dodecyl-sucrose, n-decyl-sucrose, sucrose monocaprate, sucrose monolaurate, sucrose monomyristate, and sucrose monopalmitate.

In one aspect of the invention, the surfactant is Tetradecyl-β-D-maltoside or n-dodecyl β-D-maltoside.

In one aspect of the invention, said surfactant is a poloxamer. In a further aspect of the invention, said surfactant is a poloxamer 188. In a further aspect of the invention, said surfactant is selected from the group consisting of poloxamer 407, poloxamer 124, poloxamer 181, poloxamer 182, poloxamer 237, poloxamer 331 and poloxamer 338. In a further aspect of the invention, said surfactant is a polysorbate 20 (Tween-20). In a further aspect of the invention, said surfactant is a polysorbate 80. In a further aspect of the invention, said surfactant is a Solutol HS 15. In a further aspect of the invention, said surfactant is a d-alfa-Tocopheryl polyethylene glycol 1000 succinate. In a further aspect of the invention, said surfactant a Tetradecyl-β-D-maltoside.

In one aspect of the invention, the concentration of said surfactant in the dry formulation is from about 0.1 % w/w to about 50 % w/w. In a further aspect of the invention, the concentration of said surfactant is from about 0.5 % w/w to about 10 % w/w. In a further aspect of the invention, the concentration of said surfactant is from about 1 % w/w to about 5 % w/w mg/L. In a further aspect of the invention, the concentration of said surfactant is from about 1 % w/w to about 3 % w/w. In a further aspect of the invention, the concentration of said surfactant is from about 0.1 % w/w to about 1 % w/w.

In one aspect of the invention, the surfactant is added to the said peptides before drying in a concentration between 0.1 mM and 10 mM, preferably between 0.5 mM and 5 mM, preferably between 0.5 mM and 3 mM.

In one aspect of the invention, the pharmaceutical composition comprises two different surfactants. In one aspect of the invention, said surfactants are poloxamer 188 and polysorbate 20 (Tween-20). In one aspect of the invention, at least one of the two different surfactants is an anionic surfactant.

In one aspect of the invention, the pharmaceutical composition further comprises a protamine salt, wherein said protamine salt is present in said composition in a concentration of greater than 0.25mM and wherein said composition has a pH of less than about 7.0. In the compositions of the invention, wherein a protamine salt is to be included the pH of said pharmaceutical composition or a reconstituted solution of said pharmaceutical composition is in one aspect from about pH 3.0 to about pH 6.0, in a further aspect from about pH 4.0 to about pH 5.5, in a further aspect from about pH 4.0 to about pH 5.0, in a further aspect from about pH 3.0 to about pH 4.0, and in yet a further aspect from about pH 4.0 to about pH 5.5.

In the compositions of the invention, wherein a protamine salt is to be included it is to be a protamine salt other than protamine sulphate where such salts include, but are not limited to, acetate, bromide , chloride, caproate, trifluoroacetate, HCO₃, propionate, lactate, formiate, nitrate, citrate, monohydrogenphosphate, dihydrogenphosphate, tartrate, or perchlorate salts of protamine or mixtures of any two protamine salts. "Protamine" as used herein refers to the generic name of a group of strongly basic proteins present in sperm cells in salt-like combination with nucleic acids. Normally, protamines to be used are obtained from e.g. salmon (salmine), rainbow trout (iridine), herring (clupeine), sturgeon (sturine), orspanish mackerel or tuna (thynnine) and a wide variety of salts of protamines are commercially available. Of course, it is understood that the peptide composition of a specific protamine may vary depending of which family, genera or species of fish it is obtained from. Protamine usually contains four major components, i.e. single-chain peptides containing about 30-32 residues of which about 21-22 are arginines. The N-terminal is proline for each of the four main components, and since no other amino groups are present in the sequence, chemical modification of protamine by a particular salt is expected to be homogenous in this context.

In one embodiment, the protamine salts used in the present invention are from salmon.

In another embodiment, the protamine salts used in the present invention are from herring.

In another embodiment, the protamine salts used in the present invention are from rainbow trout.

In another embodiment, the protamine salts used in the present invention are from tuna.

In another embodiment, the protamine salt is selected from the group consisting of propionate, lactate, formiate, nitrate, acetate, citrate, caproate, monohydrogenphosphate, dihydrogenphosphate salts of protamine.

In another embodiment, the protamine salt is selected from the group consisting of propionate, lactate, formiate, nitrate and acetate salts of protamine.

In another embodiment, the protamine salt is selected from acetate salts of protamine.

In the compositions of the invention, wherein a protamine salt is to be included, a surfactant may further be included. In one aspect of the invention, such a surfactant is poloxamer 188. In a further aspect of the invention, such a surfactant is selected from the group consisting of poloxamer 407, poloxamer 124, poloxamer 181, poloxamer 182, poloxamer 237, poloxamer 331 and poloxamer 338. In a further aspect, such a surfactant is polysorbate 20 (Tween-20). In a further aspect such a surfactant is a polysorbate 80. In a further aspect, such a surfactant is a Solutol HS 15. In a further aspect, such a surfactant is a d-alfa-Tocopheryl polyethylene glycol 1000 succinate. In a further aspect, such a surfactant is a Tetradecyl-β-D-maltoside.

In a further embodiment, when protamine salt is to be included in the composition of the invention it is to be a mixture of two different salts, one salt will be acetate and the other salt is selected from the group consisting of propionate, lactate, formiate, and nitrate salts of protamine. It is to be understood that when the protamine salt to be included in the formulation of the invention is to be a mixture of two different salts, the molar ratio between the two different salts may be from 0.1:1 to 1:1.

In a further embodiment, when protamine salt is to be included in the composition of the invention the insulin peptide may be selected from the group consisting of human insulin, human insulin^{B28D}, human insulin^{B28K, B29P}, and insulin glulisine. In yet a further embodiment, the insulin peptide is human insulin. In yet a further embodiment, the insulin peptide is human insulin^{B28D}. In yet a further embodiment, the insulin peptide is human insulin^{B28K, B29P}.

In one embodiment, the molar ratio of protamine salt to insulin peptide in the compositions of the invention is from about 0.5 to about 100.

In another embodiment, the molar ratio of of protamine salt to insulin peptide in the compositions of the invention is from about 0.5 to about 10.

In another embodiment, the molar ratio of protamine salt to insulin peptide in the compositions of the invention is from about 0.5 to 5.

In another embodiment, the molar ratio of protamine salt to insulin peptide in the compositions of the invention is from about 1 to 3.

In another aspect, the invention relates to a method for treatment of hyperglycemia by pulmonal or nasal administration of an effective amount of a pharmaceutical composition in particulate form comprising an amylin peptide and an insulin peptide.

In another aspect, the present invention relates to a method for treatment or prevention of type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers comprising pulmonal or nasal administration of an effective amount of a pharmaceutical composition in particulate form comprising an amylin peptide and an insulin peptide.

In another aspect, the present invention relates to a method for delaying or preventing disease progression in type 2 diabetes comprising pulmonal or nasal administration of an effective amount of a pharmaceutical composition in particulate form comprising an amylin peptide and an insulin peptide.

In another aspect, the present invention relates to a method for decreasing food intake, decreasing β-cell apoptosis, increasing β -cell function and β-cell mass, and/or for restoring glucose sensitivity to β -cells comprising pulmonal or nasal administration of an effective amount of a pharmaceutical composition in particulate form comprising an amylin peptide and an insulin peptide.

In another aspect, the present invention relates to the use of an amylin peptide and an insulin peptide for the manufacture of a pharmaceutical composition in particulate form for treatment of hyperglycemia by pulmonal or nasal administration.

In another aspect, the present invention relates to a pharmaceutical composition for use in pulmonal or nasal treatment of hyperglycemia. In another aspect, the present invention relates to the use of an amylin peptide and a meal-related insulin peptide for the manufacture of a pharmaceutical composition in particulate form for pulmonal or nasal treatment of binge eating or bulimia.

The term "polypeptide", "protein" or "peptide" is used interchangeably herein to mean a compound composed of at least five constituent amino acids connected by peptide bonds. The constituent amino acids may be from the group of the amino acids encoded by the genetic code and they may be natural amino acids which are not encoded by the genetic code, as well as synthetic amino acids. Natural amino acids which are not encoded by the genetic code are e.g. hydroxyproline, γ-carboxyglutamate, ornithine, phosphoserine, D-alanine and D-glutamine. Synthetic amino acids comprise amino acids manufactured by chemical synthesis, i.e. D-isomers of the amino acids encoded by the genetic code such as D-alanine and D-leucine, Aib (α-aminoisobutyric acid), Abu (α-aminobutyric acid), Tle (tert-butylglycine), β-alanine, 3-aminomethyl benzoic acid, anthranilic acid.

The production of polypeptides and peptides is well known in the art. Polypeptides or peptides may for instance be produced by classical peptide synthesis, e.g. solid phase peptide synthesis using t-Boc or Fmoc chemistry or other well established techniques, see e.g. Greene and Wuts, "Protective Groups in Organic Synthesis", John Wiley & Sons, 1999. The polypeptides or peptides may also be produced by a method which comprises culturing a host cell containing a DNA sequence encoding the (poly)peptide and capable of expressing the (poly)peptide in a suitable nutrient medium under conditions permitting the expression of the peptide. For (poly)peptides comprising non-natural amino acid residues, the recombinant cell should be modified such that the non-natural amino acids are incorporated into the (poly)peptide, for instance by use of tRNA mutants.

Said amylin and insulin compounds can be co-formulated either by mixing 2 powders containing each of the active pharmaceutical ingredients or by processing a solution containing both pharmaceutical ingredients into particles of suitable size by e.g. spray drying, freeze drying and micronization or the like.

Dry mixing of powder ingredients consist basically of applying three dimensional shear forces into a powder bed. The powder bed is then mixed long enough to permit true randomization of particles, which ideally should be maintained during handling and storage with minimal segregation. Segregation of powders can arise due to differences in physico-chemical and mechanical properties of the constituent particles such as size, density, morphology and surface energy.

Segregation can be mitigated by mixing particles with relatively similar physico-chemical and mechanical properties. Amylin and insulin powders can be prepared by spray drying the peptides from solutions containing surfactant which will preferential coat the surface of the spray dried particles resulting in similar levels of surface energy. Alternatively, the powder may be formulated as a loose granulate, such as a nucleus agglomerate or as spherical pellets obtained by e.g. wet granulation or tumbling granulation.

Mixing can be performed in a rotary vessel type such as a cube mixer, v-type mixer, tubula mixer or by a stationary vessel type with mechanical agitation such as an intensive mixer or gas flow agitation (fluid bed mixer), or by co-milling of premixes/granulates of the powder ingredients.

Geometric dilution can be applied if smaller quantities of a powder constituents are to be mixed into a larger bulk. This is achieved by first mixing the smaller constituent with an equal amount of diluent, mixing, then adding a larger amount of diluent equal to the mix, mixing ect., until the desired dilution and mixing has been obtained.

An ordered mixture, alternatively known as adhesive or interactive mixture, can be achieved when fine drug particle are attached by adhesion forces to the surface of coarser excipient particles (typical 60-800 µm). In this way higher degree of order is achieved relative to random mixtures on the basis of homogeneity indices.

The homogeneity of the mixture can monitored by HPLC assay of multiple samples taken from different locations in the powder mixture or alternatively be in-line monitored during mixing by near infrared spectroscopy.

In one aspect of the invention the pharmaceutical composition according to the invention is prepared by a process for spray-drying, freeze drying or vacuum drying a peptide solution, wherein
a) A peptide solution is obtained by mixing a peptide with water optionally comprising excipients and adjusting the pH of the peptide solution to become acidic, and
b) Spray-drying the peptide solution.
In a further aspect of the invention the pharmaceutical composition according to the invention is prepared by process for producing a spray-dried peptide from a peptide solution comprising:
a) Selecting a target pH for the spray-dried peptide,
b) Selecting a target pH for the peptide solution,
c) Providing an aqueous phase,
d) Adding a peptide,
e) Optionally adding excipients,
f) Adjusting the pH with a non volatile base or acid to the target pH for the spray-dried peptide,
g) Adjusting the pH with a volatile acid to the target pH of the peptide solution to be spray-dried,
h) Spray-drying the peptide solution.
wherein the steps d, e, f and g can be carried out in any order while stirring continuously.
In one aspect the peptide solution is adjusted with a volatile acid and a non volatile acid.

In one aspect the peptide solution is obtained at a temperature below about 8°C, below about 6°C, below about 5°C, below about 4°C, below about 3°C, below about 2°C or below about 1°C.

In one aspect the freezing point of the water is depressed and the peptide solution is obtained at a temperature below 0°C.

In one aspect the peptide solution has a pH below about 6.0, below about 5.5, below about 5.0, below about 4.9, below about 4.8, below about 4.7 or below about 4.5.

The tendency for the said amylin to fibrillate is less pronounced when the pH of the peptide solution is between about 2 and about 6.0, between about 2 and about 5.5, between about 2 and about 5.0, between about 2 and about 4.5.

In one aspect of the invention the peptide is added to an aqueous solution. The aqueous solution can be pure water or it can contain excipients or an acid. In one aspect of the invention the pH of the peptide solution is adjusted with an acid solution comprising a non volatile acid. The non volatile acid can be selected from the group consisting of nitric acid, hydrochloric acid, sulfuric acid, phosphoric acid, citric acid and their salts or a combination hereof.

In one aspect of the invention the pH of the peptide solution is adjusted with an acidic solution comprising a volatile acid. For example the volatile acid can be acetic acid, formic acid, triflouroacetic acid and their salts or a combination hereof.

In one aspect of the invention the pH of the peptide solution is adjusted with an acidic solution comprising a volatile acid and a non volatile acid. In one aspect the pH of the peptide solution is adjusted with hydrochloric acid to the target pH for the spray-dried peptide and with acetic acid to obtain the selected pH for the peptide solution to be spray-dried.

In one aspect of the invention, the volatile acid is used to adjusts the pH of the peptide solution with at least about 0.5 pH unit, at least about 0.7 pH unit, or at least about 0.9 pH unit or at least about 1.1 pH unit, or at least about 1.3 pH unit or at least about 1.5 pH unit.

In one aspect of the invention the peptide solution comprises phenol. In one aspect the peptide solution comprises at least about 2 moles of phenol per mole of peptide, or at least about 3 moles of phenol per mole of peptide or at least about 4 moles of phenol per mole of peptide. In one aspect the peptide solution comprises about 4 moles of phenol per mole of insulin. In one aspect the peptide solution comprises about 4 moles of phenol per mole of insulin and the peptide solution comprises zinc. When the peptide solution comprises insulin, the phenol stabilises the insulin hexamer.

In one aspect of the invention the peptide solution comprises zinc. The zinc content of the peptide solution comprising insulin can be in the range of about 2 to about 4 zinc ions per insulin hexamer, or the zinc content can be in the range of about 2.1 to about 3 zinc ions per insulin hexamer or the zinc content can be in the range of about 2.2 to about 2.7 zinc ions per insulin hexamer. The zinc content of the peptide solution comprising insulin can be about 2.3 zinc ions per insulin hexamer.

In one aspect of the invention the peptide solution comprises a buffer, a detergent, a stabilizer, a protease inhibitor, a flavour, a carrier, an absorption protacting agent, a bulking agent or an agent improving the flowing properties or a penetration enhancer.

In one aspect of the invention the peptide solution comprises glycylglycine. When glycylglycine is added to a peptide solution, the peptide dissolves more rapidly. In one aspect glycylglycine is added to an aqueous solution comprising insulin. The concentration of glycylglycine in the solution will be between about 4 mM and about 200 mM.

In one aspect of the invention the method for spray-drying a peptide solution a target pH for the spray-dried peptide is determined. The pH of the peptide solution to be spray-dried is above the target pH of the spray-dried peptide. The pH of the peptide solution may be at least about 0.5 pH units above the pH of the spray-dried peptide. The pH of the peptide solution may be at least about 0.7, at least about 0.9, at least about 1.1, at least about 1.3 or at least about 1.5, or at least about 2.0, or at least about 2.5 pH units above the pH of the of the spray-dried peptide.

In one aspect of the invention the pH of the spray dried peptide is between about 6.0 and about 8.5, between about 6.2 and about 8.4, between about 6.4 and about 8.3, between about 6.6 and about 8.2, between about 6.8 and about 8.1, between about 7.0 and about 8.0, between about 7.2 and about 7.9, between about 7.4 and about 7.8 or between about 7.6 and about 7.7.

In one aspect of the invention the peptide solution is spray dried to obtain a water content below about 10%. The water content may be below about 6%, below about 4%, below about 2% or below about 1% calculated on/measured by loss on a drying test (gravimetric).

The pharmaceutical composition of this invention can be administered by nasal or pulmonal delivery.

The pharmaceutical composition of this invention may be administered by inhalation in a dose effective manner to increase circulating insulin levels and amylin levels and/or to lower circulating glucose levels. Such administration can be effective for treating disorders such as diabetes or hyperglycaemia. Achieving effective doses of insulin requires administration of an inhaled dose of e.g. spray-dried insulin of more than about 5 µg/kg to about 250 µg/kg. Achieving effective doses of amylin requires administration of an inhaled dose of e.g. spray-dried Symlin of more than about 0.5 µg/kg to about 50 µg/kg. A therapeutically effective amount can be determined by a knowledgeable practitioner, who will take into account factors including insulin and/or amylin level, blood glucose levels, the physical condition of the patient, the patient's pulmonary status, or the like.

The composition according to the invention may be delivered by inhalation to achieve rapid absorption thereof. Administration by inhalation can result in pharmacokinetics comparable to subcutaneous administration of insulin. Inhalation of the composition according to the invention leads to a rapid rise in the level of circulating insulin and amylin followed by a rapid fall in blood glucose levels. Different inhalation devices typically provide similar pharmacokinetics when similar particle sizes and similar levels of lung deposition are compared.

The composition according to the invention may be delivered by any of a variety of inhalation devices known in the art for administration of a therapeutic agent by inhalation.

The composition according to the invention may be delivered by a dry powder inhaler (DPI). Dry Powder Inhalers are inhalers capable of delivering a powdered form of medication directly into the lung. There are a several desirable features of an inhalation device for administering the composition according to the invention. For example, delivery by the inhalation device is advantageously reliable, reproducible, and accurate. The inhalation device should deliver small particles, for example particles less than about 10 µm, for example about 1-5 µm, for good respirability.

There are two main types of DPIs, namely the passive ones and the active ones. In passive inhalers all the energy required for de-agglomerating the powder and transferring the powder to the lungs is provided by the breathing of a user or patient. Furthermore release of the drug from passive devices is synchronized by the patient's inhalation. In active inhalers there is an additional source of energy to help to de-agglomerate and aerosolize the powder such as e.g. the Nektar Pulmonary Inhaler (Nektar), Aspirair® (Vectura) or Microdose DPI (MicroDose).

Most powder inhalers are of the passive type, such as e.g. the Turbohaler^{™} (Astra-Zeneca), AIR DPI (Alkermes) and Inhalator® (Boehringer Ingelheim), where the powder is inhaled by the patient without the aid of an additional energy source. The problem with passive inhalers is that the inhalable fraction, or the proportion of powder that actually enters the lungs, is largely dependant on the breathing of the patient. The de-agglomeration of the powder and hence the inhalable fraction is a function of the flow rate of inhaled air through the device, and therefore, varies greatly from patient to patient.

Dry powder inhalers are subdivided into single dose devices such as e.g. the Aerolizer® (Novartis) and Cyclohaler® (Pharmachemie) and multi-dose inhalers. Multi-dose inhalers are further subdivided into pre-metered types where the doses are stored individually in blisters or capsules such as e.g. Diskus® (GSK) and Flowcaps® (Hovione) or the like and into reservoir based inhalers such as e.g. the Novolizer® (MEDA) where the powder dose is metered in the device.

The disadvantage of single-dose DPIs is that individual doses or cartridges must be loaded before use. This problem is overcome by multi dose pre-metered inhalers, such as the Diskus®, which contains 1 month's supply of doses. Multi dose pre-metered inhalers have the additional advantage that the single doses are metered under strict factory conditions and the powder can quite easily be isolated from e.g. humidity in the atmosphere.

Yet another inhaler principle is the Otsuka DPI (Otsuka Pharmaceutical Co)., Ltd, Tokyo, Japan]) disperses a freeze-dry cake containing the active drugs by inspiratory air flow of the patient during use.

Fixed combination therapy, multi-dose DPI's such as the Duohaler® (Innovata Plc) is an alternative to co-formulation. It has two separate reservoirs or to blister bands which can feed two separate formulations into separate metering chambers from which the formulations are delivered to the patient in the same breath. Simultaneous administration of two or more separate overcomes co-formulation issues such as segregation and physical/chemical incompatibility.

In one aspect of the invention, the pharmaceutical composition is administered in a dual chamber inhaler device.

In another aspect of the invention, the pharmaceutical composition is administered as two or more discrete powders of insulin peptide and amylin peptide by a fixed dose combination therapy inhaler.

The particle size e.g. as measured as the mean aerodynamic diameter of the composition according to the invention delivered by an inhalation device is important with respect to the ability of active agent to make it into the lungs, and into the lower airways or alveoli. The composition of this invention can be formulated so that at least about 10% of the insulin peptide and amylin peptide delivered is deposited in the lung, for example about 10 to about 20%, or more. It is known that the maximum efficiency of pulmonary deposition for mouth breathing humans is obtained with a mean aerodynamic diameter of about 2 µm to about 3 µm. Pulmonary deposition decreases substantially when particle sizes are above about 5 µm. Particle sizes below about 1 µm may cause pulmonary deposition to decrease, and it becomes difficult to deliver particles with sufficient mass to be therapeutically effective. In one aspect of the invention, particles of the composition delivered by inhalation have a mean aerodynamic diameter less than about 10 µm, for example in the range of about 1 µm to about 5 µm. The formulation of the composition is selected to yield the desired mean aerodynamic diameter in the chosen inhalation device.

Advantageously for administration as a dry powder, the composition according to the invention is prepared in a particulate form with a mean aerodynamic diameter of less than about 10 µm, for example about 1 to about 5 µm. The dry powder is largely composed of particles produced so that a majority of the particles have a size in the desired range. Advantageously, at least about 50% of the dry powder is made of particles having a mean aerodynamic diameter less than about 10 µm.

Pharmaceutical compositions according to the invention comprising a spray-dried or freeze dried insulin peptide and amylin peptide for administration from a dry powder inhaler typically include a finely divided dry powder containing the spray-dried or freeze dried peptide, but the powder can also include a bulking agent, carrier, excipient, another additive, or the like. Additives can be included in a dry powder formulation of the insulin and amylin peptide, for example, to dilute the powder as required for delivery from the particular powder inhaler, to facilitate processing of the formulation, to provide advantageous powder properties to the formulation, to facilitate dispersion of the powder from the inhalation device, to stabilize the formulation (for example, antioxidants or buffers), to provide taste to the formulation, or the like. Advantageously, the additive does not adversely affect the patient's airways. The spray-dried peptide can be mixed with an additive at a molecular level or the solid formulation can include particles of the peptide conjugate mixed with or coated on particles of the additive. Typical additives include mono-, di-, and polysaccharides; sugar alcohols and other polyols, such as, for example, lactose, glucose, raffinose, melezitose, lactitol, maltitol, trehalose, sucrose, mannitol, starch, or combinations thereof; surfactants, such as sorbitols, diphosphatidyl choline, or lecithin; or the like. Typically an additive, such as a bulking agent, is present in an amount effective for a purpose described above, often at about 50% to about 99% by weight of the formulation. Additional agents known in the art for formulation of a peptide such as insulin analog peptide can also be included in the formulation.

The peptide solution may optionally be combined with pharmaceutical carriers or excipients which are suitable for respiratory and pulmonary administration. Such carriers may serve simply as bulking agents when it is desired to reduce the insulin and/or amylin concentration in the powder which is being delivered to a patient, but may also serve to enhance the stability of the compositions and to improve the dispersability of the powder within a powder dispersion device in order to provide more efficient and reproducible delivery of the acitive ingredient and to improve handling characteristics such as flowability and consistency to facilitate manufacturing and powder filling.

Suitable carrier materials or bulking agents may be in the form of an amorphous powder, a crystalline powder, or a combination of amorphous and crystalline powders. Suitable materials include carbohydrates, e.g., monosaccharides such as fructose, galactose, glucose, D-mannose, sorbose, and the like; disaccharides, such as lactose, trehalose, cellobiose, and the like; cyclodextrins, such as 2-hydroxypropyl--cyclodextrin; and polysaccharides, such as raffinose, maltodextrins, dextrans, and the like; (b) amino acids, such as glycine, arginine, aspartic acid, glutamic acid, cysteine, lysine, and the like; (c) organic salts prepared from organic acids and bases, such as sodium citrate, sodium ascorbate, magnesium gluconate, sodium gluconate, tromethamine hydrochloride, and the like; (d) peptides and proteins, such as aspartame, human serum albumin, gelatin, and the like; (e) alditols, such as mannitol, xylitol, and the like. A preferred group of carriers includes lactose, trehalose, raffinose, maltodextrins, glycine, sodium citrate, tromethamine hydrochloride, human serum albumin, and mannitol.

Such bulking agents may be combined with the peptide prior to spray drying, i.e., by adding the carrier material to the peptide solution or the aqueous solution which is prepared for spray drying. In that way, the carrier material will be formed simultaneously with and as part of the peptide particles.

The carrier may be formed by spray drying together with the peptide, the peptide will be present in each individual particle at a weight percent in the range from 1% to 95%, preferably from 20% to 80%. The remainder of the particle will primarily be bulking agent (typically being from 5% to 95%, usually being from 20% to 80% by weight), but may also include buffer(s) and may include other components as described above.

Typically, the carriers will be separately prepared in a dry powder form and combined with the dry powder peptide by blending. The separately prepared powder carriers will usually be crystalline (to avoid water absorption), but might in some cases be amorphous or mixtures of crystalline and amorphous. The size of the carrier particles may be selected to improve the flowability of the peptide powder, typically being in the range from 25µm to 100µm. Carrier particles in this size range will generally not penetrate into the alveolar region of the lung and will often separate from the peptide(s) in the delivery device prior to inhalation. Thus, the particles which penetrate into the alveolar region of the lung will consist essentially of peptide(s) and buffer. A preferred carrier material is crystalline mannitol having a size in the above-stated range.

A spray including the spray-dried peptide of this invention can be produced by forcing a suspension or solution of peptide conjugate through a nozzle under pressure. The nozzle size and configuration, the applied pressure, and the liquid feed rate can be chosen to achieve the desired output and particle size. An electrospray can be produced, for example, by an electric field in connection with a capillary or nozzle feed. Advantageously, particles of insulin and amylin peptide (either in separate particles or co-formulated in the same particle) delivered by a sprayer have a mean aerodynamic diameter less than about 10 µm, for example in the range of about 1 µm to about 5 µm.

Compositions of this invention suitable for use with a sprayer will typically include peptides in an aqueous solution at a concentration of about 1 mg to about 60 mg of peptide conjugate per ml of solution. The composition can include agents such as a buffer, a bulking agent, a surfactant, and, for example zinc. The formulation can also include an excipient or agent for stabilization of the crystals, such as a buffer, a bulk protein, or a carbohydrate. Bulk proteins useful in formulating spray-dried peptide conjugates include albumin, protamine, or the like. Typical carbohydrates useful in formulating peptide conjugates include sucrose, mannitol, lactose, trehalose, glucose, or the like. The crystal formulation can also include a surfactant, which can reduce or prevent surface-induced aggregation of the insulin conjugate caused by atomization of the solution in forming an aerosol. Various conventional surfactants can be employed, such as polyoxyethylene fatty acid esters and alcohols, and polyoxyethylene sorbitol fatty acid esters. Amounts will generally range between about 0.001 and about 4% by weight of the formulation.

In a further aspect of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative aspect of the invention.

Examples of suitable buffers are sodium acetate, glycylglycine, HEPES (4-(2-hydroxyethyl)-1-piperazineethanesulfonic acid) and sodium phosphate.

For nasal administration an absorption enhancer may be added to the pharmaceutical composition according to the invention,

The use of an absorption enhancer may be useful, as the peptide alone is poorly absorbed through the lung. The enhancer used can be any of a number of compounds which act to enhance absorption through the layer of epithelial cells lining the lower respiratory tract, and into the adjacent pulmonary vasculature. The enhancer can accomplish this by any of several possible mechanisms:
(1) Enhancement of the paracellular permeability of a peptide by inducing structural changes in the tight junctions between the epithelial cells.
(2) Enhancement of the transcellular permeability of a peptide by interacting with or extracting protein or lipid constituents of the membrane, and thereby perturbing the membrane's integrity.
(3) Interaction between enhancer and peptide which increases the solubility of the peptide in aqueous solution. This may occur by preventing formation of insulin aggregates (dimers, trimers, hexamers), or by solubilizing peptide molecules in enhancer micelles.
(4) Decreasing the viscosity of, or dissolving, the mucus barrier lining the alveoli and passages of the lung, thereby exposing the epithelial surface for direct absorption of the peptide.

Enhancers may function by only a single mechanism set forth above, or by two or more. An enhancer which acts by several mechanisms is more likely to promote efficient absorption of a peptide than one which employs only one or two.

For example, surfactants are a class of enhancers which are believed to act by all four mechanisms listed above. Surfactants are amphiphilic molecules having both a lipophilic and a hydrophilic moiety, with varying balance between these two characteristics. If the molecule is very lipophilic, the low solubility of the substance in water may limit its usefulness. If the hydrophilic part overwhelmingly dominates, however, the surface active properties of the molecule may be minimal. To be effective, therefore, the surfactant must strike an appropriate balance between sufficient solubility and sufficient surface activity.

Another surfactant property that may be of importance is the net charge of the surfactant at the pH value in the lung (approximately 6.8). At pH 6.8, some peptides have a negative net charge. This will result in an electrostatic repulsion between molecules, which will in turn prevent aggregation and thereby increase the solubility. If the surfactant also is negatively charged, it can interact with the peptide by, for example, hydrophobic interactions, and additional repulsion among the peptide molecules will occur. In such case an anionic surfactant will possess the additional advantage (compared to those having neutral or net positive charge at physiological pH) of enhancing absorption by helping stabilize the peptide in the monomeric state.

An enhancer useful in the methods of the invention will combine effective enhancement of peptide absorption with (1) lack of toxicity in the concentrations used and (2) good powder properties, i.e., lack of a sticky or waxy consistency in the solid state. Toxicity of a given substance can be tested by standard means, such as by the MTT assay, for example as described in Int. J. Pharm., 65 (1990), 249-259. The powder properties of a given substance may be ascertained from published data on the substance, or empirically.

One very promising type of enhancer is the salt of a fatty acid such as the sodium salt of saturated fatty acids of carbon chain length 10 (i.e., sodium caprate), 12 (sodium laurate) and 14 (sodium myristate). The potassium and lysine salts of capric acid may also be used in the method of the invention. If the carbon chain length is shorter than about 10, the surface activity of the surfactant may be too low, and if the chain length is longer than about 14, decreased solubility of the fatty acid salt in water limits its usefulness.

Most preferably in the present invention the substance which enhances the absorption of peptide in the lower respiratory tract is sodium caprate.

Different counterions may change the solubility of the saturated fatty acid salt in water, such that an enhancer having a carbon length other than 10-14 would prove even more advantageous than the enhancers specifically mentioned hereinabove. Salts of unsaturated fatty acids may also be useful in the present invention since they are more water soluble than salts of saturated fatty acids, and can therefore have a longer chain length than the latter and still maintain the solubility necessary for a successful enhancer of peptide absorption.

All of the bile salts and bile salt derivatives tested (sodium salts of ursodeoxycholate, taurocholate, glycocholate, and taurodihydrofusidate) effectively enhance peptide absorption in the lung.

Phospholipids has also been tested as enhancers. It was found that a single-chain phospholipid (lysophospatidylcholine) was an effective enhancer, while two double-chain phospholipids (dioctanoylphosphatidylcholine and didecanoylphosphatidylcholine) were not. This may be explained by the fact that the double-chain phospholipids are much less soluble in water than their single-chain counterparts; however, it is reasonable to expect that double-chain phospholipids of shorter chain length, having greater water-solublility than their longer chain counterparts, will be of use as enhancers in the present invention so that both single- and double-chain phospholipids may be used.

One glycoside, octylglucopyranoside, has been suggested as an enhancer in the present invention. Other alkyl glycosides, such as thioglucopyranosides and maltopyranosides would also be expected to exhibit absorption enhancing properties in the methods of the present invention.

The cyclodextrins and derivatives thereof effectively enhance nasal absorption, and may function similarly in the lung. Dimethyl-β-cyclodextrin has been suggested and was found to have an absorption enhancing effect.

Other potentially useful surfactants are sodium salicylate, sodium 5-methoxysalicylate, and the naturally occurring surfactants such as salts of glycyrrhizine acid, saponin glycosides and acyl carnitines.

For ionic enhancers (e.g., the anionic surfactants described above), the nature of the counterion may be important. The particular counterion selected may influence the powder properties, solubility, stability, hygroscopicity, and local/systemic toxicity of the enhancer or of any formulation containing the enhancer. It may also affect the stability and/or solubility of the peptide with which it is combined. In general, it is expected that monovalent metallic cations such as sodium, potassium, lithium, rubidium, and cesium will be useful as counterions for anionic enhancers. Ammonia and organic amines form another class of cations that is expected to be appropriate for use with anionic enhancers having a carboxylic acid moiety. Examples of such organic amines include ethanolamine, diethanolamine, triethanolamine, 2-amino-2-methylethylamine, betaines, ethylenediamine, N,N-dibensylethylenetetraamine, arginine, hexamethylenetetraamine, histidine, N-methylpiperidine, lysine, piperazine, spermidine, spermine and tris(hydroxymethyl)aminomethane.

Since effective enhancement of peptide absorption in the lung was observed for a number of the enhancers tested, it is expected that many more will be found which also function in this manner. Starch microspheres effectively enhance the bioavailability of peptide delivered via the nasal membranes and were tested as an enhancer in the methods of the invention. Although they proved to be of little use for delivery via the pulmonary route in the animal model utilized herein, it is thought that this was mainly due to technical difficulties which, if overcome, may lead to successful delivery via the pulmonary route.

Chelators are a class of enhancers that are believed to act by binding calcium ions. Since calcium ions help maintain the dimensions of the space between cells and additionally reduce the solubility of a peptide, binding of these ions would in theory both increase the solubility of peptides, and increase the paracellular permeability of peptides.

### Proportions of Peptide and Enhancer

The relative proportions of peptide and enhancer may be varied as desired. Sufficient enhancer must be present to permit efficient absorption of the inhaled peptide; however, the amount of enhancer should be kept as low as possible in order to minimize the risk of adverse effects caused by the enhancer. Although each particular peptide/enhancer combination must be tested to determine the optimal proportions, it is expected that to achieve acceptable absorption of the peptide, more than 10% of the peptide/enhancer mixture must be enhancer; for most types of enhancers, the proportion of enhancer should be more than 15% or more than 20% and will preferably be between 25% and 50%. The preferred ratio for each peptide/enhancer (or peptide/enhancer/diluent) combination can be readily determined by one of ordinary skill in the art of pharmacology by standard methods, based on such criteria as efficient, consistent delivery of the optimal dosage, minimization of side effects, and acceptable rate of absorption.

No further ingredients are needed for the action of the preparation, but may be included if desired. For example, the amount of powder which constitutes a single dose of a given peptide/surfactant combination could be increased (e.g., for use in an inhaler apparatus which by design requires a large powder volume per dose) by diluting the powder with pharmaceutically acceptable diluents. Other additives may be included to facilitate processing or to improve the powder properties or stability of the preparation. A flavouring agent could be added so that the proportion of the powder which is inevitably deposited in the mouth and throat would serve to give the patient positive feedback that a dose had been delivered from the inhaler device. Any such additive should have the following properties: (a) it is stable and does not disadvantageously affect the stability of the peptide and enhancer; (b) it does not disadvantageously interfere with absorption of the peptide; (c) it has good powder properties, as that term is understood in the pharmaceutical arts; (d) it is not hygroscopic; and (e) it has no adverse effects in the airways in the concentrations used. Useful types of such additives include mono-, di-, and polysaccharides, sugar alcohols, and other polyols: for example, lactose, glucose, raffinose, melezitose, lactitol, maltitol, trehalose, sucrose, mannitol, and starch. As reducing sugars such as lactose and glucose have a tendency to form complexes with proteins, non-reducing sugars such as raffinose, melezitose, lactitol, maltitol, trehalose, sucrose, mannitol and starch may be preferred additives for use in the present invention. Such additives may constitute anywhere from 0% (i.e., no additive) to nearly 100% of the total preparation.

In a preferred embodiment, this invention provides a therapeutic preparation of a pharmaceutically active peptide and a substance which enhances the absorption of said peptide in the lower respiratory tract, which preparation is in the form of a dry powder preparation suitable for inhalation of which at least 50% by mass consists of (a) particles having a diameter of less than about 10 microns or (b) agglomerates of said particles; in another preferred embodiment, the invention provides a therapeutic preparation comprising a pharmaceutically active peptide, a substance which enhances the absorption of peptide in the lower respiratory tract, and a pharmaceutically acceptable carrier, which preparation is in the form of a dry powder suitable for inhalation of which at least 50% by mass consists of (a) particles having a diameter of less than about 10 microns, or (b) agglomerates of said particles; and in a further preferred embodiment this invention provides a therapeutic preparation comprising active compounds (A) a pharmaceutically active peptide and (B) a substance which enhances the absorption of said peptide in the lower respiratory tract, wherein at least 50% of the total mass of active compounds (A) and (B) consists of particles having a diameter of less than about 10 microns, and a pharmaceutically acceptable carrier, which preparation is in the form of a dry powder preparation suitable for inhalation in which an ordered mixture may be formed between the active compounds and the pharmaceutically acceptable carrier.

In one embodiment of the invention the pharmaceutical composition according to the invention is stable for more than 6 weeks of usage and for more than 3 years of storage.

In another embodiment of the invention the pharmaceutical composition according to the invention is stable for more than 4 weeks of usage and for more than 3 years of storage.

In a further embodiment of the invention the pharmaceutical composition according to the invention is stable for more than 4 weeks of usage and for more than two years of storage.

In an even further embodiment of the invention the pharmaceutical composition according to the invention is stable for more than 2 weeks of usage and for more than two years of storage.

In one embodiment, the pharmaceutical composition according to the invention is used for the preparation of a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, stroke, coronary heart disease and other cardiovascular disorders, inflammatory bowel syndrome, dyspepsia and gastric ulcers.

In another embodiment, the pharmaceutical composition according to the invention is used as a medicament for delaying or preventing disease progression in type 2 diabetes.

In another embodiment, the pharmaceutical composition according to the invention is used as a medicament for decreasing food intake, decreasing β-cell apoptosis, increasing β-cell function and β-cell mass, and/or for restoring glucose sensitivity to β-cells.

In one embodiment of the invention, the pharmaceutical composition according to the invention is for use as a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers or for delaying or preventing disease progression in type 2 diabetes or for decreasing food intake, decreasing β-cell apoptosis, increasing β-cell function and β-cell mass, and/or for restoring glucose sensitivity to β-cells, is provided.

In a further embodiment of the invention, a method for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers or for delaying or preventing disease progression in type 2 diabetes or for decreasing food intake, decreasing β-cell apoptosis, increasing β-cell function and β-cell mass, and/or for restoring glucose sensitivity to β-cells, the method comprising administering to a patient in need of such treatment an effective amount for such treatment of the pharmaceutical composition according to the invention, is provided.

The treatment with the pharmaceutical composition according to the invention may also be combined with a second or more pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are: GLP-1 and GLP-1 derivatives and analogues, GLP-2 and GLP-2 derivatives and analogues, Exendin-4 and Exendin-4 derivatives and analogues, amylin and amylin derivatives and analogues, sulphonylureas, biguanides, meglitinides, glucosidase inhibitors, glucagon antagonists, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents as HMG CoA inhibitors (statins), compounds lowering food intake, RXR agonists and agents acting on the ATP-dependent potassium channel of the β-cells; Cholestyramine, colestipol, clofibrate, gemfibrozil, lovastatin, pravastatin, simvastatin, probucol, dextrothyroxine, neteglinide, repaglinide; β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR β agonists; histamine H3 antagonists, gastrin and gastrin analogues and derivatives.

Further embodiments according to the invention:
1. A pharmaceutical composition in particulate form comprising an amylin peptide and an insulin peptide.
2. The pharmaceutical composition according to embodiment 1, wherein the amylin peptide and the insulin peptide are co-formulated.
3. The pharmaceutical composition according to embodiment 1, wherein the amylin peptide and the insulin peptide are formulated in two separate powders.
4. The pharmaceutical composition according to embodiment 3, wherein the amylin peptide powder and the insulin peptide powder are in the form of a physical mixture.
5. The pharmaceutical composition according any one of the embodiments 1-4 for pulmonal administration.
6. The pharmaceutical composition according any one of the embodiments 1-5, which has a mean aerodynamic diameter of 0.5 to 10 µm.
7. The pharmaceutical composition according any one of the embodiments 1-6, which has a mean aerodynamic diameter of 1 to 5 µm.
8. The pharmaceutical composition according any one of the embodiments 1-7, which has a mean aerodynamic diameter of 1 to 4 µm.
9. The pharmaceutical composition according any one of the embodiments 1-8, which has a mean aerodynamic diameter of 1 to 3 µm.
10. The pharmaceutical composition according any one of the embodiments 1-9, which further comprises a bulking agent.
11. The pharmaceutical composition according any one of the embodiments 1-4 for nasal administration.
12. The pharmaceutical composition according to embodiment 11, which has a mean aerodynamic diameter of 10 to 800 µm.
13. The pharmaceutical composition according any one of the embodiments 11-12, which has a mean aerodynamic diameter of 20 to 100 µm.
14. The pharmaceutical composition according any one of the embodiments 11-13, which has a mean aerodynamic diameter 50 to 100 µm.
15. The pharmaceutical composition according any one of the embodiments 11-14, which has a mean aerodynamic diameter 1 to 3 µm.
16. The pharmaceutical composition according any one of the embodiments 1-9, which further comprises an absorption enhancer.
17. The pharmaceutical composition according to any one of the embodiments 1-16, wherein said insulin peptide is human insulin, an analog of human insulin, a derivative of human insulin or a derivative of a human insulin analog.
18. The pharmaceutical composition according to any one of the embodiments 1-17, wherein the insulin peptide is a meal-related insulin peptide.
19. The pharmaceutical composition according to embodiment 18, wherein said meal-related insulin peptide has a time action of less than 4 hours.
20. The pharmaceutical composition according to any one of the embodiments 1-19, wherein said insulin peptide is human insulin.
21. The pharmaceutical according to any one of the embodiments 1-17, wherein said insulin peptide is a human insulin analog.
22. The pharmaceutical composition according to embodiment 21, wherein said human insulin analog is human insulin^{B28D}.
23. The pharmaceutical composition according to embodiment 21, wherein said human insulin analog is human insulin^{B28K, B29P}.
24. The pharmaceutical composition according to embodiment 21, wherein said human insulin analog is human insulin^{B3K, B29E}.
25. The pharmaceutical composition according to embodiment 21, wherein said human insulin analog is human insulin ^{desB30}.
26. The pharmaceutical composition according to embodiment 21, wherein said insulin peptide is a derivative of a human insulin analog.
27. The pharmaceutical composition according to embodiment 21, comprising two different insulin peptides.
28. The pharmaceutical composition according to any one of the embodiments 1-27, wherein said amylin peptide is amylin, an amylin analog or an amylin agonist.
29. The pharmaceutical composition according to embodiment 28, wherein said amylin peptide is human amylin.
30. The pharmaceutical composition according to embodiment 28, wherein said amylin peptide is ^{25,28,29}Pro- h-amylin.
31. The pharmaceutical composition according to embodiment 28, wherein said amylin peptide is human amylin methylated in position 24 and 26.
32. The pharmaceutical composition according to any one of the embodiments 1-31, wherein said insulin peptide is human insulin and said amylin peptide is ^{25,28,29}Pro- h-amylin.
33. The pharmaceutical composition according to any one of the embodiments 1-31, wherein said insulin peptide is human insulin^{B28D} and said amylin peptide is ^{25,28,29}Pro- h-amylin.
34. The pharmaceutical composition according to any one of the embodiments 1-31, wherein said insulin peptide is human insulin^{B3K,B29E} and said amylin peptide is ^{25,28,29}Pro- h-amylin.
35. The pharmaceutical composition according to embodiment 34, wherein the concentration of human insulin^{B3K, B29E} is in the range from about 0.36 mg/mL to about 4.0 mg/mL.
36. The pharmaceutical composition according to any one of the embodiments 1-31, wherein said insulin peptide is Asp^{B28} human insulin and said amylin peptide is ^{25,28,29}Pro- h-amylin.
36. The pharmaceutical composition according to any one of the embodiments 1-31, wherein said amylin peptide is ^{25,28,29}Pro- h-amylin and said insulin peptide is Asp^{B28} human insulin is present in a molar ratio 1:5 to1:1.
37. The pharmaceutical composition according to any one of the embodiments 1-36 for use as a medicament in the treatment of hyperglycemia.
38. A method for treatment of hyperglycemia comprising oral administration of an effective amount of the pharmaceutical composition as defined in any of the embodiments 1-37.
39. Use of the pharmaceutical composition according to any one of the embodiments 1-36 for administration in a dual chamber inhaler device.
40. Use of the pharmaceutical composition according to any one of the embodiments 1-36 for administration as two or more discrete powders of insulin peptide and amylin peptide by a fixed dose combination therapy inhaler.

It should be understood that any suitable combination of therapeutically active polypeptides in the pharmaceutical composition according to the invention and optionally one or more further pharmacologically active substances are considered to be within the scope of the present invention.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law).

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

### EXAMPLES

### EXAMPLE 1

100 mg insulin aspart was dissolved in 10 ml ice cold demineralised water. pH of the solution was adjusted from 7.5 to 2.8 with 1 M acetic acid. 100 mg pramlintide acetate was dissolved in the ice cold insulin aspart solution. The pH of the insulin aspart-pramlintide solution was adjusted from pH 3.2 to pH 2.8 with 1 M acetic acid and the volume was adjusted to 33 ml with demineralised water. The resulting concentration of both pramlintide acetate and insulin aspart was 3.0 mg/ml. Hence, the dry matter concentration was in total 6 mg/ml.

The solution was spray dried on a Büchi B-290 mini spray dryer (Büchi, Labortechnik AG Flawil, Switzerland) equipped with a 0.7 mm co-current two-fluid nozzle. The insulin human solution was atomised into hot air stream in a drying chamber at a liquid feed rate of 2 ml/min and atomising air flow of 600-800 liter/hour.

The drying air had an inlet temperature of 150°C and a drying air flow rate of 35 m³/hour. The outlet temperature was approximately 70°C.

Solid microparticles were captured by a cyclone connected to the drying chamber and then gathered and stored at dry conditions.

The aerodynamic diameter of the powder was determined using a TSI Model 3321 Aerodynamic Particle Sizer® (APS™) spectrometer with a model 3433 Small-Scale Powder Disperser. The mean mass aerodynamic diameter was 1.66 µm.

### EXAMPLE 2

### Powder I:

200 mg pramlintide acetate was dissolved in 20 ml ice cold demineralised water. pH was adjusted from 3.2 to 5.2 with 0.1 M NaOH and the volume was adjusted to 33 ml with demineralised water.

The solution was spray dried on a Büchi B-290 mini spray dryer (Büchi, Labortechnik AG Flawil, Switzerland) equipped with a 0.7 mm co-current two-fluid nozzle. The insulin human solution was atomised into hot air stream in a drying chamber at a liquid feed rate of 2 ml/min and atomising air flow of 600-800 liter/hour.

The drying air had an inlet temperature of 150°C and a drying air flow rate of 35 m³/hour. The outlet temperature was approximately 64°C.

Solid microparticles were captured by a cyclone connected to the drying chamber and then gathered and stored at dry conditions.

The aerodynamic diameter of powder I was determined using a TSI Model 3321 Aerodynamic Particle Sizer® (APS™) spectrometer with a model 3433 Small-Scale Powder Disperser. The mean mass aerodynamic diameter was 1.66 µm.

### Powder 2:

400 mg insulin aspart was dissolved in 40 ml ice cold demineralised water. pH of the solution was adjusted from 7.3 to 7.7 with 0.1 M NaOH and the volume was adjusted to 66 ml with demineralised water.

The solution was spray dried on a Büchi B-290 mini spray dryer (Büchi, Labortechnik AG Flawil, Switzerland) equipped with a 0.7 mm co-current two-fluid nozzle. The insulin human solution was atomised into hot air stream in a drying chamber at a liquid feed rate of 2 ml/min and atomising air flow of 600-800 liter/hour.

The drying air had an inlet temperature of 150°C and a drying air flow rate of 35 m³/hour. The outlet temperature was approximately 61°C.

Solid microparticles were captured by a cyclone connected to the drying chamber and then gathered and stored at dry conditions.

The aerodynamic diameter of powder I was determined using a TSI Model 3321 Aerodynamic Particle Sizer® (APS™) spectrometer with a model 3433 Small-Scale Powder Disperser. The mean mass aerodynamic diameter was 1.70 µm.

Next, 100 mg of Symlin powder (I) and 100 mg of powder (II) is mixed by spatulation using a sandwich technique. The powder mixture is sieved and mixed again and finally sieved for a second time. 10 random samples

The homogeneity is assessed by HPLC analysis of 5 samples taken from different locations in the powder mixture. The powder mixture is accepted as homogeneous and used for further testing when the relative standard deviation (RSD) is less than 10%.

### EXAMPLE 3:

For pulmonary delivery the PennCentury™ DP-4 dry powder insufflator (Penn-Century Inc) modified for pigs was used in combination with the PennCentury™ adjustable air pump, model AP-1 (Penn-Century Inc). The powder is weighed into the sample chamber of the device and manually emitted from the device by using the AP-1 adjustable pump.

The pigs were fasted for approximately 18 h before the experiment. Anaesthesia was induced by injecting Domitor IV (1 mg/mL, 0.4 mL/pig), followed by slow IV infusion of Propofol (10 mg/ml) until the sufficient depth of anaesthesia was obtained, approximately 10-15 mL/pig in total. Atropin (1 mg/ml, 0.5 mL/pig) was injected i.m. to stabilize cardiac parameters and to decrease bronchial secretions. After anaesthesia (and) the pigs were intubated with an endotracheal tube size 8.0 mm. Via an L-piece and the endotraceal tube the pigs were connected to a respirator, with respiration frequency set to 10/min and tidal volume to 300 mL/breath to mimic deep inhalations in humans. The delivery tube of the device was introduced through the endotracheal tube, and pramlintide/insulin aspart powder was delivered intra-tracheally above the tracheal bifurcation. Each pig was given 5-10 air sprays in the beginning of each of 5-10 consecutive inhalations. Immediately after dosing the pigs were given an IM injection of Antisedan (5 mg/mL, 0.4 mL/pig) to counteract the effect of Domitor and were taken back to their pens where they were allowed to wake up from the anaesthesia. The majority of blood samples were thus taken in conscious animals.

Central venous blood samples were collected in EDTA-coated tubes at the following time points relative to dosing: -10, 0, 5, 10, 20, 30, 40, 50, 60, 75, 90, 105, 120, 150, 180, 210, 240 and 300 minutes. The blood was centrifuged, and the resulting plasma stored at -20 oC until analysis.

Insulin aspart was analyzed using an in-house LOCI sandwich immunoassay with two different antibodies. The antibody (HUI-018) coated to Acceptor beads is a monoclonal mice antibody directed against ordinary insulin. The second biotinylated antibody (X14-6-F34) is another monoclonal mice antibody, which is specific against Insulin Aspart. The lower limit of detection was 1 pM. Pramlintide was analyzed using a commercially available human amylin ELISA kit modified for porcine samples (Linco Research, cat. no. EZHA-52K). The lower limit of detection was 1 pM.

Estimated mean dose were 5.3±0.7 nmol/kg for insulin aspart and 8.8 nmol/kg for Symlin.

Absolute bioavailability was 10.3±4 % for insulin aspart and 5.1±3.1 % for symlin, and the respective values for tmax were 81±42 min and 40±18 min.

## Claims

1. A pharmaceutical composition in particulate form comprising an amylin peptide and an insulin peptide.

2. The pharmaceutical composition according to claim 1, wherein the amylin peptide and the insulin peptide are co-formulated.

3. The pharmaceutical composition according to claim 1, wherein the amylin peptide and the insulin peptide are formulated in two separate powders.

4. The pharmaceutical composition according to claim 3, wherein the amylin peptide powder and the insulin peptide powder are in the form of a physical mixture.

5. The pharmaceutical composition according any one of the claims 1-4, which is for pulmonary administration.

6. The pharmaceutical composition according any one of the claims 1-5, which has a mean aerodynamic diameter of 0.5 to 10 µm.

7. The pharmaceutical composition according to any one of the claims 1-6, wherein said insulin peptide is human insulin.

8. The pharmaceutical according to any one of the claims 1-7, wherein said insulin peptide is a human insulin analog.

9. The pharmaceutical composition according to claim 8, wherein said human insulin analog is human insulin^{B28D}.

10. The pharmaceutical composition according to claim 9, wherein said amylin peptide is ^{25,28,29} Pro- h-amylin.

11. The pharmaceutical composition according to any one of the claims 1-10, wherein said insulin peptide is human insulin and said amylin peptide is ^{25,28,29} pro- h-amylin.

12. The pharmaceutical composition according to any one of the claims 1-10, wherein said insulin peptide is human insulin^{B28D} and said amylin peptide is ^{25,28,29} Pro- h-amylin.

13. The pharmaceutical composition according to any one of the claims 1-10, wherein said amylin peptide is ^{25,28,29} Pro- h-amylin and said insulin peptide is Asp^{B28} human insulin is present in a molar ratio 1:5 to1:1.

14. The pharmaceutical composition according to any one of the claims 1-13 for use as a medicament in the treatment of hyperglycemia.

15. A method for treatment of hyperglycemia comprising oral administration of an effective amount of the pharmaceutical composition as defined in any of the claims 1-14.
